# EUROPEAN PATENT SPECIFICATION

(11) **EP 3 230 907 B1**
(45) Date of publication and mention of the grant of the patent: **26.09.2018**
(21) Application number: 15813912.1
(22) Date of filing: 30.11.2015
(51) Int. Cl.: G06F 19/00, G16H 10/60

(54) **SYSTEM AND METHOD FOR UNIFORMLY CORRELATING UNSTRUCTURED ENTRY FEATURES TO ASSOCIATED THERAPY FEATURES**
SYSTEM UND VERFAHREN ZUR GLEICHFÖRMIGEN KORRELATION UNSTRUKTURIERTER EINGABEFUNKTIONEN ZU ASSOZIIERTEN THERAPIEFUNKTIONEN
SYSTÈME ET PROCÉDÉ POUR METTRE EN CORRÉLATION UNIFORMÉMENT DES CARACTÉRISTIQUES D'ENTRÉE NON STRUCTURÉES À DES CARACTÉRISTIQUES DE THÉRAPIE ASSOCIÉES

(30) Priority: 09.12.2014 US 201462089336 P
(43) Date of publication of application: 18.10.2017
(73) Proprietor: Koninklijke Philips N.V., 5656 AE Eindhoven (NL)
(72) Inventor: SHARIFI SEDEH, Reza, NL-5656 AE Eindhoven (NL); FARRI, Oladimeji Feyisetan, NL-5656 AE Eindhoven (NL); ZHU, Xianshu, NL-5656 AE Eindhoven (NL); JIA, Yugang, NL-5656 AE Eindhoven (NL); ELGORT, Daniel Robert, NL-5656 AE Eindhoven (NL)
(74) Representative: de Haan, Poul Erik
(86) International application number: PCT/IB2015/059203
(87) International publication number: WO 2016/092411

(56) References cited:
- EP-A1- 2 669 812
- WO-A2-00/72452
- WO-A2-2007/024617
- US-A1- 2011 113 049

## Description

### BACKGROUND

### 1. Field

The present disclosure pertains to a system and method to uniformly correlate unstructured entry features in unstructured therapy entries to structured entry features in structured therapy information. Uniformly correlated entry features are beneficial for secondary use in clinical and patient safety-related research.

### 2. Description of the Related Art

It is well known that computerized provider order entry (CPOE) systems have been adopted across the national healthcare landscape. The CPOE systems provide a platform for expedited prescription, reduction of medication errors, and creation of large electronic drug databases for clinical research. The CPOE systems do not ensure drug data interoperability. The information entered into the CPOE systems is not normalized.

WO 00/72452 A2 discloses an apparatus including an input device including a plurality of differentiated selections for input information, whereby said input information can include unstructured information; a computing element capable of selecting a protocol in response to said unstructured information, said protocol including a sequence of structured triggers and responses; an output device including a plurality of differentiated presentations for said triggers, whereby a set of corresponding responses, entered at said input device, in response to said triggers presented at said output device, include structured information in response to said unstructured information.

### SUMMARY

The invention is defined in the appended claims. Aspects, embodiments and examples disclosed of the present disclosure which do not fall under the scope of the appended claims do not form part of the invention and are merely provided for illustrative purposes.

Accordingly, one or more aspects of the present disclosure relate to a system configured to uniformly correlate unstructured entry features in unstructured therapy entries to associated therapy features in structured therapy information. The system comprising one or more physical computer processors configured by computer readable instructions to: obtain unstructured therapy entries, the unstructured therapy entries including a first unstructured therapy entry; identify unstructured entry features in the individual unstructured therapy entries; and correlate the identified unstructured entry features in the unstructured therapy entries to corresponding associated therapy features, such correlation being based on contextual information associated with the unstructured therapy entries in which the unstructured entry features are included, such that, responsive to identification of a first unstructured entry feature in the first unstructured therapy entry, the first unstructured entry feature is correlated to a corresponding associated therapy feature based on contextual information associated with the first unstructured therapy entry, the contextual information associated with the first unstructured therapy entry including one or more of a syntax of the first unstructured therapy entry, a creator of the first unstructured therapy entry, and/or a format of the first unstructured therapy entry.

Yet another aspect of the present disclosure relates to a method for uniformly correlating unstructured entry features in unstructured therapy entries to associated therapy features in structured therapy information with a correlation system. The system comprising one or more physical computer processors. The method comprising: obtaining, with the one or more physical computer processors, unstructured therapy entries, the unstructured therapy entries including a first unstructured therapy entry; identifying, with the one or more physical computer processors, unstructured entry features in the individual unstructured therapy entries; and correlating, with the one or more physical computer processors, the identified unstructured entry features in the unstructured therapy entries to corresponding associated therapy features, such correlation being based on contextual information associated with the unstructured therapy entries in which the unstructured entry features are included, such that, responsive to identification of a first unstructured entry feature in the first unstructured therapy entry, the first unstructured entry feature is correlated to a corresponding associated therapy feature based on contextual information associated with the first unstructured therapy entry, the contextual information associated with the first unstructured therapy entry including one or more of a syntax of the first unstructured therapy entry, a creator of the first unstructured therapy entry, and/or a format of the first unstructured therapy entry.

Still another aspect of present disclosure relates to a system configured to uniformly correlate unstructured entry features in unstructured therapy entries to associated therapy features in structured therapy information. The system comprises means for obtaining unstructured therapy entries, the unstructured therapy entries including a first unstructured therapy entry; means for identifying unstructured entry features in the individual unstructured therapy entries; and means for correlating the identified unstructured entry features in the unstructured therapy entries to corresponding associated therapy features, such correlation being based on contextual information associated with the unstructured therapy entries in which the unstructured entry features are included, such that, responsive to identification of a first unstructured entry feature in the first unstructured therapy entry, the first unstructured entry feature is correlated to a corresponding associated therapy feature based on contextual information associated with the first unstructured therapy entry, the contextual information associated with the first unstructured therapy entry including one or more of a syntax of the first unstructured therapy entry, a creator of the first unstructured therapy entry, and/or a format of the first unstructured therapy entry.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosure.

### BRIEF DESCRIPTION OF THE DRAWINGS

FIG. 1 is an illustration of a system configured to uniformly correlate unstructured entry features in unstructured therapy entries to associated therapy features in structured therapy information.
FIG. 2 illustrates correlation of unstructured entry features to corresponding associated therapy features.
FIG. 3 illustrates one embodiment of a refinement application system.
FIG. 4 illustrates a natural language processing (NLP) and machine learning engine for drug database preprocessing.
FIG. 5 illustrates a method for uniformly correlating unstructured entry features to associated therapy features.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

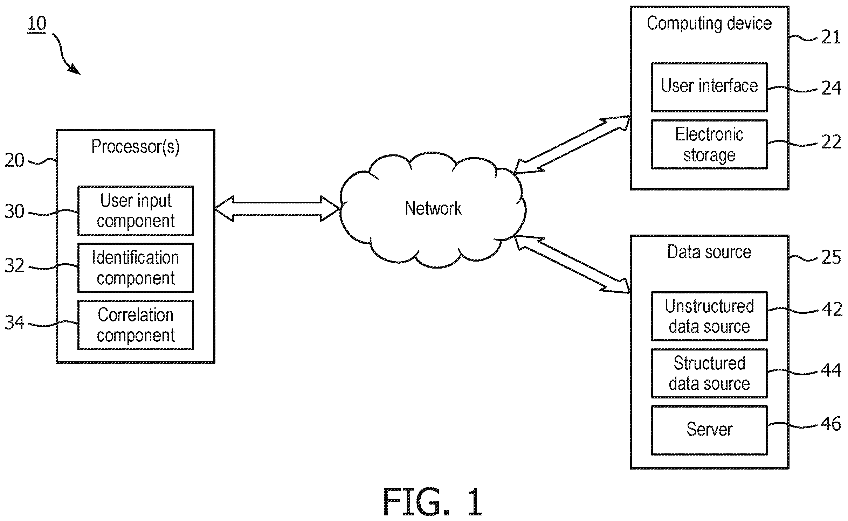

FIG. 1 schematically illustrates a system 10 configured to uniformly correlate unstructured entry features in unstructured therapy entries to associated therapy features in structured therapy information. Issues with computerized provider order entry (CPOE) systems and drug data interoperability continually persist due to variability in local (clinician-generated) preferences for drug names and/or standard coding systems, resulting in limited normalization of entries in drug databases and potential inaccuracies in research findings derived from such data. Widely used standard terminologies like the Food and Drug Administration's NDC (National Drug Code) can be difficult to implement and manage as the codes are created by individual drug suppliers, not a universal governing organization. The First Data Bank MedKnowledge, National Drug Data File (NDDF), and the Hierarchical Ingredient Code List (HICL) terminologies provide viable alternatives to the NDC. However, due to errors in data entry and the use of ambiguous abbreviations for medications within healthcare environments, mismatches in names and HICL or NDDF codes are often rife in drug databases. These issues adversely impact the secondary use of drug databases in clinical care-related research.

System 10 is configured to identify and/or analyze unstructured therapy entry features and correlate them to corresponding associated therapy features in structured information. Such identification and/or analysis may include textual analysis with natural language processing (NLP), machine learning, and/or other techniques and/or models. Machine learning models (e.g., naive Bayes and/or other models) learn from a set of previously identified and correlated (to associated therapy features) unstructured entry features and then apply learned rules to classify subsequently identified unstructured entry features. When there are errors and/or other inconsistencies in the unstructured therapy entries (e.g. misspellings and/or inconsistent abbreviations) the performance of typical machine learning systems declines. System 10 may combine machine learning techniques (e.g. Naive Bayes and/or others) with pre-processing and/or disambiguation approaches (e.g., NLP-based textual analysis) to correlate unstructured therapy entry features with corresponding associated therapy features (e.g., classify unstructured therapy entry features based on HICL or NDDF codes) with enhanced uniformity and/or accuracy. Facilitating classification of unstructured therapy entry features with enhanced uniformity enhances interoperability by normalizing unstructured therapy entry features to large structured therapy-related information databases. Facilitating classification of the unstructured therapy entry features with enhanced uniformity makes the data more beneficial for secondary use in clinical and patient safety-related research. For example, system 10 may facilitate standardized coding of drug names in pharmaceutical/clinical databases. In some embodiments, system 10 includes one or more of a computing device 21, a data source 25, a processor 20, and/or other components.

Computing device 21 may be configured such that a user may access system 10 via computing device 21. Computing device 21 may include one or more of a user interface 24, electronic storage 22, a processor 20, and/or other components. In some embodiments, computing device 21 may be configured to communicate with one or more external data sources 25, processor 20, and/or other components of system 10. Computing device 21 may be configured to communicate with data sources 25, processor 20, and/or other components of system 10 according to peer-to-peer architecture, client/server architecture, and/or other architectures. Byway of a non-limiting example, a given computing device 21 may include one or more of a desktop computer, a laptop computer, a handheld computer, a tablet computing platform, a NetBook, a smartphone, and/or other computing platforms. Computing device 21 may include communication lines, and/or ports to enable the exchange of information with a network, other computing platforms (e.g., one or more other computing devices 21), and/or other devices. In some embodiments, communication between computing device 21, processor 20, data source 25, and/or other components of system 10 may be wireless and/or via wires. For example, computing device 21 may communicate with processor 20 and/or data source 25 wirelessly via a Wi-Fi network, via Bluetooth® technology, and/or other wireless technology. In some embodiments, computing device 21 may communicate with user processor 20 and/or data source 25 via a wired USB connection, for example. In some embodiments, computing device 21 may include processor 20 and/or data source 25.

User interface 24 is configured to provide an interface between system 10 and a provider 12, and/or other users through which provider 12 and/or other users may provide information to and receive information from system 10. This enables data, cues, results, unstructured therapy entries, and/or instructions and any other communicable items, collectively referred to as "information," to be communicated between a user and one or more data sources 25, processors 20, and/or other components of system 10.

Examples of interface devices suitable for inclusion in user interface 24 comprise a keypad, buttons, switches, a keyboard, knobs, levers, a display screen, a touch screen, speakers, a microphone, an indicator light, an audible alarm, a printer, a tactile feedback device, and/or other interface devices. In some embodiments, user interface 24 comprises a plurality of separate interfaces. In some embodiments, user interface 24 comprises at least one interface that is provided integrally with data source 25, processor 20, and/or other components of system 10.

It is to be understood that other communication techniques, either hardwired or wireless, are also contemplated by the present disclosure as user interface 24. For example, the present disclosure contemplates that user interface 24 may be integrated with a removable storage interface provided by electronic storage 22. In this example, information may be loaded into system 10 from removable storage (e.g., a smart card, a flash drive, a removable disk, etc.) that enables the user(s) to customize the implementation of system 10. Other exemplary input devices and techniques adapted for use with system 10 as user interface 24 comprise, but are not limited to, an RS-232 port, RF link, an IR link, modem (telephone, cable or other). In short, any technique for communicating information with system 10 is contemplated by the present disclosure as user interface 24.

User interface 24 is configured to facilitate Computerized Provider Order Entry (CPOE) and/or other information entry and/or selection. This may include entry and/or selection of unstructured therapy information (unstructured therapy entries that include therapy entry features) via user interface 24. In some embodiments, a provider (e.g. doctors, nurses, users, and/or other providers) inputs unstructured therapy information (e.g., a textual therapy entry for a specific patient that describes the treatment provided to and/or prescribed for the patient that includes drug names, quantities, treatment times, frequencies, and/or other entry features) with user interface 24 and/or other components. System 10 is configured such that this information is stored in data source 25 and/or in other devices. As described above, unstructured (e.g., textual) therapy entries may include drug units, doses, abbreviations, drug/therapy information, and/or other information and/or features. In some embodiments, unstructured therapy entries may include misspellings, abbreviations, abbreviations specific to the provider who enters the information, ambiguous terms, and/or other information. In some embodiments, computing device 21 may be configured to facilitate storage of such unstructured therapy entries in electronic storage 22, facilitate communication of unstructured therapy entries to data source 25, and/or take other actions with the unstructured therapy entries.

Electronic storage 22 comprises electronic storage media that electronically stores information. The electronic storage media of electronic storage 22 may comprise one or both of system storage that is provided integrally (i.e., substantially non-removable) with computing device 21 and/or removable storage that is removably connectable to system 10 via, for example, a port (e.g., a USB port, a firewire port, etc.) or a drive (e.g., a disk drive, etc.). Electronic storage 22 may comprise one or more of optically readable storage media (e.g., optical disks, etc.), magnetically readable storage media (e.g., magnetic tape, magnetic hard drive, floppy drive, etc.), electrical charge-based storage media (e.g., EPROM, RAM, etc.), solid-state storage media (e.g., flash drive, etc.), and/or other electronically readable storage media. Electronic storage 22 may store software algorithms, information determined by processor 20, information received from a user via user interface 24, and/or other information that enables system 10 to function properly. Electronic storage 22 may be (in whole or in part) a separate component within system 10, or electronic storage 22 may be provided (in whole or in part) integrally with one or more other components of system 10 (e.g., within computing device 21).

Data source 25 is configured to electronically store therapy records, unstructured therapy entries, structured therapy information (e.g., associated therapy features, associations of associated therapy features (e.g., groups of related associated therapy features), previously determined correlations between unstructured therapy entry features and associated therapy features, and/or other information. In some embodiments, data source 25 may include an unstructured data source 42, a structured data source 44, and/or other sources of information. In some embodiments, data source 25 may be and/or include a server 46 and/or other components.

Unstructured data source 42 is configured to store unstructured therapy information. The unstructured therapy information includes therapy records and/or other information. Therapy records may include unstructured therapy entries, contextual information, and/or other information. Unstructured therapy entries are obtained and/or received from various sources (e.g., various computing devices 21). Unstructured therapy entries include unstructured therapy entry features and/or other information. Unstructured data source 42 is configured to store unstructured therapy entries received via computing device 21 and/or other sources of unstructured therapy information. Unstructured data source 42 is configured to store contextual information related to and/or characterizing therapy (e.g., medical therapy and/or other therapy). Such information may be entered and/or selected by a provider. Such information may include one or more of a name or names of the provider(s), a geographic location of the patient and/or the treatment facility, a hospital name and/or location, a facility type, a job title of the provider (e.g. doctor, nurse, pharmacist, etc.); words, sentences, abbreviations, syntax, formatting, etc. that connect various drug names, quantities, treatment frequencies, and/or other information in an unstructured therapy entry; and/or other information. Unstructured therapy entries may include unstructured therapy entry features (e.g., provider name, provider job title, treatment facility name/location, drug names, quantities, treatment frequency, etc.) and/or other information. Unstructured therapy entries may include abbreviations, ambiguous words, misspellings, inconsistent units, and/or other non-uniformities. These non-uniformities may be artefacts of user entry, institutional practices and/or defaults, inadvertent propagation of redundant errors within health information systems, and/or be caused by other factors.

Structured data source 44 is configured to store associated therapy features, associations of associated therapy features, previously determined correlations of unstructured entry features to associated therapy features of structured information, and/or other information. Associated therapy features may include drug names, drug ingredients, quantities (e.g., dose), identification codes, sequence codes, chemical compositions, routes of administration, and/or other therapeutic information. Associations of associated therapy features may include sets of associated therapy features, classes of associated therapy features (e.g., within a set), and/or other groups of related associated therapy features. In some embodiments, structured data source 44 may be and/or include one or more databases of structured information. In some embodiments, the structured information stored in structured data source 44 may have a hierarchical arrangement and/or other formats. For example, structured data source 44 may include NDC, First Databank MedKnowledge (NDDF), RxNorm, HICL, , and/or other databases. By way of a non-limiting example, in a database such as an NDDF database, the associated therapy features may correspond to drug names, for example. In this database (for example), the associations of associated therapy features may include a drug class that includes the several drug names. This drug class may be part of a larger set of drug classes of the same type (e.g., another association of associated therapy features).

In some embodiments, data source 25 may be and/or include one or more servers 46. Servers 46 may be configured to store unstructured therapy entries, unstructured therapy entry features, associated therapy entries, previously determined correlations between unstructured therapy entry features and associated therapy features, and/or other information (e.g., servers 46 may include unstructured data source 42 and/or structured data source 44); communicate with computing device 21, processor 20, and/or other devices; and/or perform other functions. In some embodiments, processor 20 may be included in servers 46 and/or the functions of processor 20 described below may be performed by servers 46. In some embodiments, server 46 may be a single server. In some embodiments, server 46 may be and/or include multiple servers operating together with a cloud configuration to perform the functions described herein. Server 46 may include electronic storage, one or more processors (e.g., processor 20), and/or other components. Servers 46 may include communication lines, or ports to enable the exchange of information with a network and/or other computing platforms. Illustration of servers 46 in FIG. 1 as a single entity is not intended to be limiting of servers 46 and/or data source 25. Servers 46 may include a plurality of hardware, software, and/or firmware components operating together to provide the functionality attributed herein to servers 46. For example, servers 46 may be implemented by a cloud of computing platforms operating together as servers 46.

Processor(s) 20 are configured to provide information processing capabilities in system 10. As such, processor 20 may include one or more of a digital processor, an analog processor, a digital circuit designed to process information, an analog circuit designed to process information, a state machine, and/or other mechanisms for electronically processing information. Although processor 20 is shown in FIG. 1 as a single entity, this is for illustrative purposes only. In some embodiments, processor 20 may comprise a plurality of processing units. These processing units may be physically located within the same device, or processor 20 may represent processing functionality of a plurality of devices operating in coordination.

As shown in FIG. 1, processor 20 is configured to execute one or more computer program components. Processor 20 may include one or more of a user input component 30, an identification component 32, a correlation component 34, and/or other components. Processor 20 may be configured to execute components 30, 32, 34, and/or other computer processing components by software; hardware; firmware; some combination of software, hardware, and/or firmware; and/or other mechanisms for configuring processing capabilities on processor 20. It should be appreciated that although components 30, 32, 34, and , are illustrated in FIG. 1 as being co-located within a single processing unit, in embodiments in which processor 20 includes multiple processing units, one or more of components 30, 32, 34, and/or other computer processing components may be located remotely from the other components. The description of the functionality provided by the different components 30, 32, 34, and/or other computer processing components described below is for illustrative purposes, and is not intended to be limiting, as any of components 30, 32, 34, and/or other computer processing components may provide more or less functionality than is described. For example, one or more of components 30, 32, 34, and/or other computer processing components may be eliminated, and some or all of its functionality may be provided by other ones of components 30, 32, 34, and/or other computer processing components. As another example, processor 20 may be configured to execute one or more additional components that may perform some or all of the functionality attributed below to one of components 30, 32, 34, and/or other computer processing components.

User input component 30 is configured to obtain unstructured therapy entries from computing device 21 (e.g., after entry and/or selection by a provider and/or other users of system 10), from unstructured data source 42, and/or from other sources. User input component 30 may obtain the unstructured therapy entries in real time or near real time from computing device 21, in one or more batches of one or more unstructured therapy entries from unstructured data source 42, and/or in other ways.

Identification component 32 is configured to identify unstructured therapy entry features in the unstructured therapy entries. In some embodiments, identification component 32 is configured to determine the context of the unstructured therapy entry features in the unstructured therapy entries. In some embodiments, identification component 32 is configured to use the contextual information in a therapy record related to and unstructured therapy entry to identify the features of an unstructured therapy entry.

Correlation component 34 is configured to correlate the identified unstructured entry features in the unstructured therapy entries to corresponding structured entry features. Correlation component 34 correlates the unstructured therapy entries to structured entry features based on contextual information associated with the individual unstructured therapy entries in which the unstructured entry features are included. For example, responsive to identification of a first unstructured therapy entry feature, the first unstructured therapy entry feature is correlated to a corresponding structured entry feature based on contextual information associated with the first unstructured therapy entry feature.

In some embodiments, correlation component 34 is configured such that correlation includes using a natural language processing (NLP) algorithm for textual analysis of the unstructured therapy entries. Using an NLP algorithm for textual analysis may include several steps such as: (a) correcting misspellings in the unstructured therapy entries, (b) disambiguating abbreviations by providing the full-length descriptions for the abbreviations, (c) identifying trade and/or common names for drugs and/or therapies mapping them to a generic name and/or description of the trade and/or common name, and/or (d) identifying and removing non-semantic (nonsense) words included in the unstructured therapy entries, and/or other steps.

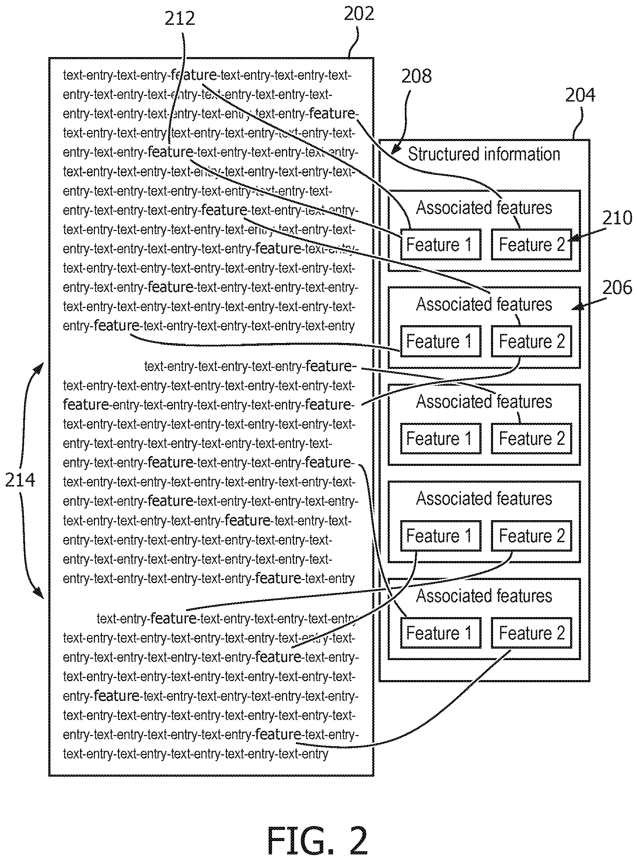

By way of a non-limiting example, FIG. 2 illustrates correlating identified unstructured entry features 212 in an unstructured therapy entry 202 of a therapy record with corresponding associated therapy features 210 of structured information 204. Structured information 204 includes associations 206 of associated therapy features 210. An identified unstructured entry feature 212 such as a drug name (e.g. Tylenol) may be correlated with one or more associated features 210 of structured information 204 (e.g. generic name acetaminophen, drug number, recommended dose, etc.) In some embodiments, correlation 208 of unstructured entry features 212 with associated therapy features 210 by correlation component 34 (FIG. 1) also correlates a particular unstructured therapy entry feature 212 with an association 206 (e.g., a class of drug and/or a drug code) of associated therapy features 210 in structured information 204 (e.g., a particular database such as an HICL drug code database). Contextual information 214 associated with the unstructured entry features 212 (e.g., syntax, creator, format, and/or other features of the unstructured therapy entry 202) is used by correlation component 34 (FIG. 1) for the correlation of unstructured entry features 212 with associated features 210. In some embodiments, correlation 208 of an unstructured entry feature 212 with a particular associated therapy feature 210 correlates, modifies, and/or changes the unstructured entry feature 212 into a structured entry feature.

Returning to FIG. 1, in some embodiments, correlation component 34 is configured such that correlation includes a training phase and an application phase. In the training phase, after identification of the unstructured therapy entry features by identification component 32, and then correlation of unstructured therapy entry features to associated therapy features by correlation component 34, the correlations may be learned by correlation component 34 using a machine learning model (e.g. a naive Bayesian estimator) and/or by other methods. Learning the correlations may comprise recognizing similar and/or the same correlations that occur one or more times for one or more unstructured therapy entry features in the unstructured therapy entries. In some embodiments, machine learning models are based on corrected drug-names and their corresponding codes (e.g. RxNorm, HICL or NDDF sequence codes and/or other nationally and/or internationally accepted coding standards.) During the training phase, (e.g., using the machine learning model), correlation component 34 determines a correlation strength for the individual correlations (e.g., a likelihood that an unstructured therapy entry feature is actually associated with an associated therapy feature that it has been correlated to). In some embodiments, based on the determined correlation strength between the unstructured entry features and the associated therapy features, correlation component 34 is then configured to determine a likelihood that a particular unstructured therapy entry feature falls within a class (e.g., an association of associated therapy features) of drugs and/or drug codes in a particular database (e.g., HICL drug code database). For example, the unstructured entry features may include therapy names and/or classification numbers, and correlation component 34 is configured to determine a first probability that the therapy names and/or classification numbers are related to a set of therapy names and/or classification numbers (e.g., an association of associated therapy features) in a database (e.g., structured information), and determine a second probability that the unstructured therapy features (e.g. names and/or classification numbers) are related to a specific information class (e.g. HICL or NDDF) of therapy names and/or classification numbers (e.g., a second association of associated therapy features).

The correlation strength and the likelihood of an unstructured therapy entry feature falling within a class of drugs and/or drug codes are used by correlation component 34 during a subsequent application phase. During the application phase, identified unstructured therapy entry features are correlated to associated therapy features based on the previous correlations during the learning phase. During the application phase, the correlations may be made based on the previously determined correlation strengths of the learned correlations. The application phase may include recognizing similar and/or the same correlations (e.g. those that were learned above) as they occur one or more times for one or more unstructured therapy entry features in the unstructured therapy entries. In some embodiments, the determined correlation strength between the entry features and the associated therapy features is used by correlation component 34 to correlate a particular unstructured therapy entry feature with an individual associated therapy feature and/or an association of associated therapy features (e.g. a class of drugs). For example the first probability (e.g. that the unstructured therapy feature is related to a associated therapy feature) and the second probability (e.g. that the unstructured therapy feature is related to a particular association of associated therapy features) can be used to determine the strength of the correlation for subsequent unstructured therapy features. During the application phase of correlation, posterior probabilities of the unstructured entry features belonging to one or more specific associations of associated therapy features (e.g., drug classes) are determined. In some embodiments, an unstructured therapy entry feature is correlated to an associated therapy feature that has the highest posterior probability of correlation. In some embodiments, the corresponding posterior probability is a reliability coefficient.

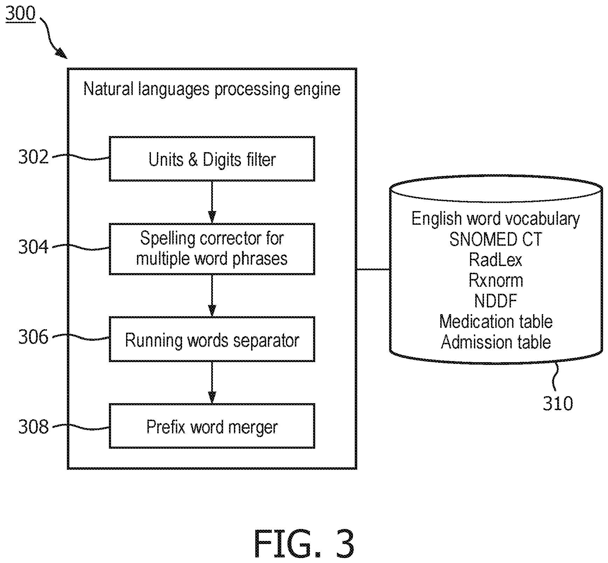

FIG. 3 illustrates natural language processing (NLP) operations performed by correlation component 34 (shown in FIG. 1). An NLP Engine 300 (e.g., correlation component 34) may normalize unstructured therapy information with units and digits filter 302, spelling corrector for multiple word phrases 304, running words separator 306, prefix word merger 308, and/or other components. Units and digits filter 302 may enable unstructured therapy entry feature (e.g. name and/or classification number) normalization. Recognizing contextual information contained in a therapy record associated with the unstructured therapy entry (e.g. units) enables units and digits filter 302 to remove the units and allows system 10 to correlate the entry features themselves.

Spelling corrector for multiple word phrases 304 includes a dictionary that is built from multiple data sources 25 (shown in FIG. 1). Spelling corrector for multiple word phrases 304 may include a database 310 that includes one or more of a general English vocabulary, SNOMED CT, RadLex, RxNorm, NDDF, HICL, proprietary medication and admission tables, and/or other multiple word dictionary phrases. In some embodiments, database 310 is indexed on both unigram and bigram entities. In some embodiments, a context-aware spell checking algorithm corrects misspellings in drug names.

Unstructured therapy entries (e.g., drug names) may comprise words that are devoid of accurate white spaces and/or punctuation. Running words separator 306 is configured to identify and reformat therapy entries in an accurate and uniform fashion. For example, the common unstructured therapy entries include the drug name "calciumgluconate" (should be "calcium gluconate") and "sodabicarb" (should be "soda bicarb"). Running words separator 306 separates words that are run together based on prefix mismatch detection and/or bigram search.

Similarly, sometimes words are given white space and/or punctuation that should not be included. Prefix word merger 308 creates an accurate and uniform method of formatting therapy entries. Because a specific documentation style for drug name entries in one database may not be uniform with other databases (or internal to the same database) some providers 12 (e.g. clinicians) add white spaces in between prefixes and/or actual terms in drug names. For example, "multivitamins" should be written as "multi vitamins". Prefix word merger 308 aims to normalize such words by joining prefix with its proceeding word in an accurate and uniform fashion.

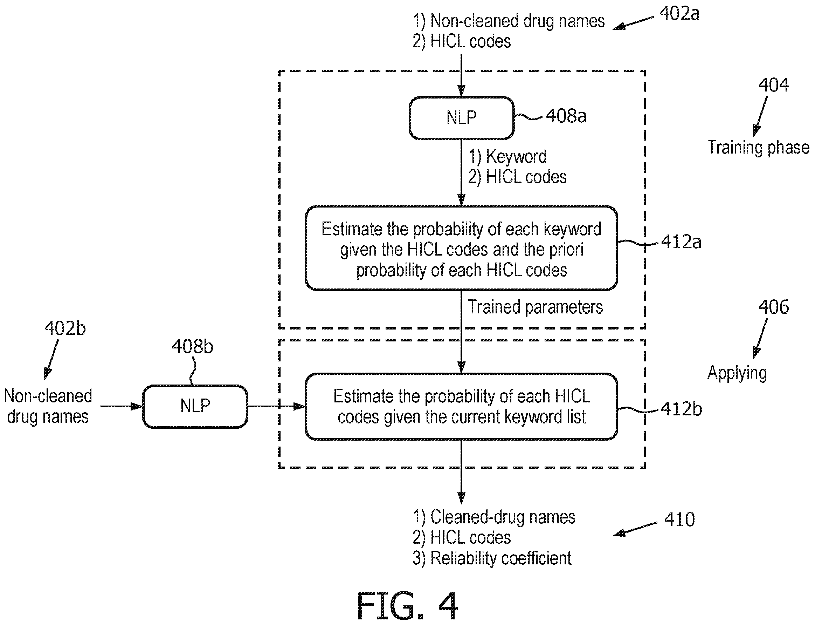

FIG. 4 illustrates implementing machine learning to train system 10 with preprocessed (e.g., by identification component 32) therapy entries. Unstructured therapy entries 402a and 402b are processed through a training phase 404 (e.g. a training phase as described above) and an application phase 406 (e.g. an application phase as described above). After the machine learning model is trained (e.g. during training phase 404) using the preprocessed drug and/or therapy names (e.g. unstructured entry features) with the NLP module 408a and correlated to the corresponding associated therapy features, the model is applied to new drug and/or therapy names (e.g. unstructured entry features) to accurately and uniformly correlate the drug/therapy features (e.g. application phase 406). In some embodiments, the naive Bayes machine learning method is used to estimate the posterior probability of the correlation of the unstructured entry features with associated therapy features from a given keyword list that have been cleaned with preprocessing (e.g. NLP module). The structured therapy features with the highest posterior probability are selected as the machine learned output 410. The posterior probability of the correlation found by system 10 is provided as a reliability coefficient and provided in the machine learned output 410.

In some embodiments, the unstructured therapy entry 402a used for training is analogous to the unstructured therapy entry 402b that is used in the application phase 406 of the machine learned correlation. In both the training phase 404 and the application phase 406 the unstructured therapy entry is preprocessed with an NLP module (408a, 408b respectively) prior to correlating the unstructured therapy entry features to associated therapy features. Correlation may entail comparing the estimated probability of each unstructured entry feature to the associated therapy feature for the trained correlation 412a as well as the application correlation 412b. This comparison generates a posterior probability of associations of associated therapy features being included in an unstructured therapy feature list. The correlations are proportional to the likelihood of each structured therapy entry in the unstructured therapy entry data being correlated to an associated therapy feature and the prior probability of each structured therapy entry being within the structured information. The parameters are computed during training phase 404 with the large collection of unstructured therapy entries with correct structured therapy entries. The parameters are then used in the application phase 406 to make the correlations between unstructured therapy entry features and associated therapy features.

For example, in some embodiments, the correlation of three unstructured features (e.g. unstructured therapy entry features) to associated therapy features within two HICL structured information classes (e.g. associations of associated therapy features) requires calculating the probability that each unstructured feature is in the HICL class (e.g., association of associated therapy features):

```
  p(HICL | featurel,feature2,feature3) ∝ p(HICL)p(featurel,feature2,feature3 | HICL)
         ≈p(HICL) p(featurel | HICL)p(feature2 | HICL)p(feature3 | HICL)
```

If only unstructured feature 1 appears in the unstructured therapy entry, the posterior probability of the unstructured therapy entry belonging to HICL class one is correlated as follows:

```
                 p(HICL=1 | feature1=1,feature2=0,feature3=0)
  ≈p(HICL=1)p(feature1=1 | HICL=1)p(feature2=0 | HICL=1)p(feature3=0 | HICL=1)
```

Four parameters of the above equation can be computed during a training phase, for example:

```
     p(feature1=1 | HICL=1) = (Total counts of HICL=1 AND feature1 appears)/
                          (Total counts of HICL=1)
```

The machine learning aspect of uniformly correlating unstructured therapy entries to structured therapy entries is designed to facilitate the secondary use of the eRI drug database in clinical research (Data Mining and Knowledge Discovery). As extension of the secondary therapy entry data use, the system may provide correlating effects for other databases of unstructured entries (e.g. eRI database to external customers). Examples of the use of therapy entries are not intended to be limiting. For example, adverse event and/or infection detection, bio surveillance performed by healthcare quality-focused and public health organizations e.g. the Center for Disease Prevention and Control (CDC), may be improved by implementation of a system to accurately and uniformly correlate large quantities of unstructured entry features to associated therapy features and/or associations of associated therapy features. Uniform standardization techniques may be useful in other areas of research, for example, in refining national drug databases or unifying clinical research CPOE documentation. The examples explained herein are illustrative only and are not intended to be limiting on the scope of this disclosure.

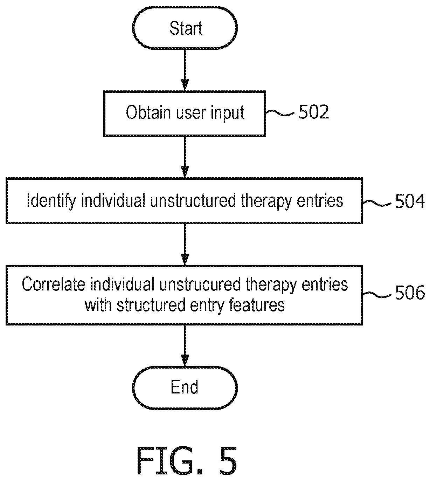

FIG. 5 illustrates a method 500 for uniformly correlating unstructured entry features in unstructured therapy entries to associated therapy features with a correlation system. The correlation system comprises one or more physical computer processors and/or other components configured by computer readable instructions to execute computer program components. The computer program components include a user input component, an identification component, a correlation component, and/or other components. The operations of method 500 presented below are intended to be illustrative. In some embodiments, method 500 may be accomplished with one or more additional operations not described, and/or without one or more of the operations discussed. Additionally, the order in which the operations of method 500 are illustrated in FIG. 5 and described below is not intended to be limiting.

In some embodiments, method 500 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 500 in response to instructions stored electronically on an electronic storage medium. The one or more processing devices may include one or more devices configured through hardware, firmware, and/or software to be specifically designed for execution of one or more of the operations of method 500.

At an operation 502 user input is obtained. User input may include unstructured therapy entries. For example, a provider may input an unstructured therapy entry (e.g. drug prescription) at a CPOE user interface. In some embodiments, operation 502 is performed by a user input component the same or similar to user input component 30 (shown in FIG. 1 and described herein).

At an operation 504 individual unstructured therapy entry features are identified. Unstructured therapy entry features are included in unstructured therapy entries input in operation 502. In some embodiments, operation 504 is performed by an identification component the same or similar to identification component 32 (shown in FIG. 1 and described herein).

At an operation 506 individual unstructured therapy entry features are correlated to associated therapy features. The identified unstructured entry features in the unstructured therapy entries are correlated to structured entry features with such correlation being based on contextual information associated with the individual unstructured therapy entries in which the unstructured entry features are included. Responsive to identification of an unstructured therapy entry, the unstructured therapy entry is correlated to a corresponding associated therapy feature based on contextual information associated with the unstructured therapy entry. Contextual information includes one or more of syntax of the first unstructured therapy entry, a creator of the first unstructured therapy entry, and/or a format of the first unstructured therapy entry. In some embodiments, correlation may include correcting misspellings in the unstructured therapy entries, providing descriptions for abbreviations in the unstructured therapy entries, removing non-semantic and/or nonsense data from the unstructured therapy entries, filtering units, correcting spelling of words, separating words, merging prefix separated words, correcting misspellings, providing descriptions, removing non-semantic and/or nonsense data, and/or other operations. In some embodiments, operation 506 is performed by a correlation component the same or similar to correlation component 34 (shown in FIG. 1 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the description provided above provides detail for the purpose of illustration based on what is currently considered to be the most practical and preferred embodiments, it is to be understood that such detail is solely for that purpose and that the disclosure is not limited to the expressly disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A system (10) configured to uniformly correlate unstructured entry features (212) in unstructured therapy entries (202) to associated therapy features (210) in structured therapy information (204), the system comprising:
one or more physical computer processors (20) configured by computer readable instructions to:
obtain unstructured therapy entries, the unstructured therapy entries including a first unstructured therapy entry;
identify unstructured entry features in the individual unstructured therapy entries; and
correlate the identified unstructured entry features in the unstructured therapy entries to corresponding associated therapy features, such correlation being based on contextual information associated with the unstructured therapy entries in which the unstructured entry features are included, such that, responsive to identification of a first unstructured entry feature in the first unstructured therapy entry, the first unstructured entry feature is correlated to a corresponding associated therapy feature based on contextual information associated with the first unstructured therapy entry, the contextual information associated with the first unstructured therapy entry including one or more of a syntax of the first unstructured therapy entry, a creator of the first unstructured therapy entry, and/or a format of the first unstructured therapy entry.

2. The system of claim 1, wherein the one or more physical computer processors are configured such that the associated therapy features comprise one or more of a drug ingredient in a Hierarchical Ingredient Code List (HICL), a drug name in a National Drug Data File (NDDF), a therapy name, or a classification number.

3. The system of claim 1, wherein the one or more physical computer processors are configured such that the unstructured entry features include one or more of an unstructured drug ingredient, an unstructured drug name, an unstructured therapy name, or an unstructured classification number, and wherein the one or more physical computer processors are configured to determine a first probability that an individual unstructured entry feature is related to a set of associated therapy features, and determine a second probability that the individual unstructured entry feature is related to a specific class of associated therapy features in the set of associated therapy features.

4. The system of claim 1, wherein the one or more physical computer processors are configured to determine a reliability factor indicative of correlation strength between the first unstructured entry feature and the corresponding associated therapy feature.

5. The system of claim 1, wherein the one or more physical computer processors are configured such that correlation includes one or more of
filtering units from the unstructured therapy entries;
correcting spelling of words in the unstructured therapy entries; separating words in the unstructured therapy entries;
merging prefix separated words in the unstructured therapy entries;
correcting misspellings in the unstructured therapy entries;
providing descriptions for abbreviations in the unstructured therapy entries; and/or
removing non-semantic and/or nonsense data from the unstructured therapy entries.

6. A method for uniformly correlating unstructured entry features (212) in unstructured therapy entries (202) to associated therapy features (210) in structured therapy information (204) with a correlation system (10), the system comprising one or more physical computer processors (20), the method comprising:
obtaining, with the one or more physical computer processors, unstructured therapy entries, the unstructured therapy entries including a first unstructured therapy entry;
identifying, with the one or more physical computer processors, unstructured entry features in the individual unstructured therapy entries; and
correlating, with the one or more physical computer processors, the identified unstructured entry features in the unstructured therapy entries to corresponding associated therapy features, such correlation being based on contextual information associated with the unstructured therapy entries in which the unstructured entry features are included, such that, responsive to identification of a first unstructured entry feature in the first unstructured therapy entry, the first unstructured entry feature is correlated to a corresponding associated therapy feature based on contextual information associated with the first unstructured therapy entry, the contextual information associated with the first unstructured therapy entry including one or more of a syntax of the first unstructured therapy entry, a creator of the first unstructured therapy entry, and/or a format of the first unstructured therapy entry.

7. The method of claim 6, wherein the associated therapy features comprise one or more of a drug ingredient in a Hierarchical Ingredient Code List (HICL), a drug name in a National Drug Data File (NDDF), a therapy name, or a classification number.

8. The method of claim 6, wherein the unstructured entry features include one or more of an unstructured drug ingredient, an unstructured drug name, an unstructured therapy name, or an unstructured classification number, and wherein the method further comprises determining a first probability that an individual unstructured entry feature is related to a set of associated therapy features, and determining a second probability that the individual unstructured entry feature is related to a specific class of associated therapy features in the set of associated therapy features.

9. The method of claim 6, further comprising determining a reliability factor indicative of correlation strength between the first unstructured entry feature and the corresponding associated therapy feature.

10. The method of claim 6, wherein correlation includes one or more of:
filtering units from the unstructured therapy entries;
correcting spelling of words in the unstructured therapy entries;
separating words in the unstructured therapy entries;
merging prefix separated words in the unstructured therapy entries;
correcting misspellings in the unstructured therapy entries;
providing descriptions for abbreviations in the unstructured therapy entries; and/or
removing non-semantic and/or nonsense data from the unstructured therapy entries.

## Patentansprüche

1. System (10), das so konfiguriert ist, dass es unstrukturierte Eintragsmerkmale (212) in unstrukturierten Therapieeinträgem (202) mit zugeordneten Therapiemerkmalen (210) in strukturierten Therapieinformationen (204) einheitlich in Übereinstimmung bringt, wobei das System umfasst:
einen oder mehrere physikalische Computerprozessoren (20), die durch computerlesbare Instruktionen konfiguriert werden, um:
unstrukturierte Therapieeinträge zu erhalten, wobei die unstrukturierten Therapieeinträge einen ersten unstrukturierten Therapieeintrag enthalten;
unstrukturierte Eintragsmerkmale in den individuellen unstrukturierten Therapieeinträgen zu identifizieren; und
die identifizierten, unstrukturierten Eintragsmerkmale in den unstrukturierten Therapieeinträgen mit entsprechenden zugeordneten Therapiemerkmalen in Übereinstimmung zu bringen, wobei eine solche Korrelation auf kontextuellen Informationen basiert, die den unstrukturierten Therapieeinträgen zugeordnet sind, in denen die unstrukturierten Eintragsmerkmale enthalten sind, so dass, in Reaktion auf die Identifikation eines ersten unstrukturierten Eintragsmerkmals in dem ersten unstrukturierten Therapieeintrag, das erste unstrukturierte Eintragsmerkmal aufgrund von kontextuellen, dem ersten unstrukturierten Therapieeintrag zugeordneten Informationen mit einem entsprechenden zugeordneten Therapiemerkmal in Übereinstimmung gebracht wird, wobei die dem ersten unstrukturierten Therapieeintrag zugeordneten, kontextuellen Informationen eine(n) oder mehrere einer Syntax des ersten unstrukturierten Therapieeintrags, eines Erstellers des ersten unstrukturierten Therapieeintrags und/oder eines Formats des ersten unstrukturierten Therapieeintrags enthalten.

2. System nach Anspruch 1, wobei der eine oder mehrere physikalische Computerprozessoren so konfiguriert sind, dass die zugeordneten Therapiemerkmale eines oder mehrere eines Arzneimittelwirkstoffes in einer Hierarchical Ingredient Code List (HICL), eines Arzneimittelnamens in einer National Drug Data File (NDDF), eines Therapienamens oder einer Klassifizierungsnummer umfassen.

3. System nach Anspruch 1, wobei der eine oder mehrere physikalische Computerprozessoren so konfiguriert sind, dass die unstrukturierten Eintragsmerkmale einen oder mehrere eines unstrukturierten Arzneimittelwirkstoffes, eines unstrukturierten Arzneimittelnamens, eines unstrukturierten Therapienamens oder einer unstrukturierten Klassifizierungsnummer enthalten, und wobei der eine oder mehrere physikalische Computerprozessoren so konfiguriert sind, dass sie eine erste Wahrscheinlichkeit ermitteln, dass ein individuelles unstrukturiertes Eintragsmerkmal auf einen Satz zugeordneter Therapiemerkmale bezogen ist, und eine zweite Wahrscheinlichkeit ermitteln, dass das individuelle unstrukturierte Eintragsmerkmal auf eine spezifische Klasse zugeordneter Therapiemerkmale in dem Satz zugeordneter Therapiemerkmale bezogen ist.

4. System nach Anspruch 1, wobei der eine oder mehrere physikalische Computerprozessoren so konfiguriert sind, dass sie einen Zuverlässigkeitsfaktor ermitteln, der für eine Korrelationsstärke zwischen dem ersten unstrukturierten Eintragsmerkmal und dem entsprechenden zugeordneten Therapiemerkmal indikativ ist.

5. System nach Anspruch 1, wobei der eine oder mehrere physikalische Computerprozessoren so konfiguriert sind, dass eine Korrelation einen oder mehrere der folgenden Schritte umfasst, wonach:
Einheiten aus den unstrukturierten Therapieeinträgen herausgefiltert werden;
die Rechtschreibung von Wörtern in den unstrukturierten Therapieeinträgen korrigiert wird;
Wörter in den unstrukturierten Therapieeinträgen getrennt werden;
getrennte Präfix-Wörter in den unstrukturierten Therapieeinträgen kombiniert werden;
Rechtschreibfehler in den unstrukturierten Therapieeinträgen korrigiert werden;
Beschreibungen für Abkürzungen in den unstrukturierten Therapieeinträgen bereitgestellt werden; und/oder
nicht-semantische und/oder sinnwidrige Daten aus den unstrukturierten Therapieeinträgen entfernt werden.

6. Verfahren, um unstrukturierte Eintragsmerkmale (212) in unstrukturierten Therapieeinträgem (202) mit zugeordneten Therapiemerkmalen (210) in strukturierten Therapieinformationen (204) mit einem Korrelationssystem (10) einheitlich in Übereinstimmung zu bringen, wobei das System einen oder mehrere physikalische Computerprozessoren (20) umfasst, wobei gemäß dem Verfahren:
mit Hilfe des einen oder mehrerer physikalischer Computerprozessoren unstrukturierte Therapieeinträge erhalten werden, wobei die unstrukturierten Therapieeinträge einen ersten unstrukturierten Therapieeintrag enthalten;
mit Hilfe des einen oder mehrerer physikalischer Computerprozessoren unstrukturierte Eintragsmerkmale in den individuellen unstrukturierten Therapieeinträgen identifiziert werden; und
mit Hilfe des einen oder mehrerer physikalischer Computerprozessoren die identifizierten, unstrukturierten Eintragsmerkmale in den unstrukturierten Therapieeinträgen mit entsprechenden zugeordneten Therapiemerkmalen in Übereinstimmung gebracht werden, wobei eine solche Korrelation auf kontextuellen Informationen basiert, die den unstrukturierten Therapieeinträgen zugeordnet sind, in denen die unstrukturierten Eintragsmerkmale enthalten sind, so dass, in Reaktion auf die Identifikation eines ersten unstrukturierten Eintragsmerkmals in dem ersten unstrukturierten Therapieeintrag, das erste unstrukturierte Eintragsmerkmal aufgrund von kontextuellen, dem ersten unstrukturierten Therapieeintrag zugeordneten Informationen mit einem entsprechenden zugeordneten Therapiemerkmal in Übereinstimmung gebracht wird, wobei die dem ersten unstrukturierten Therapieeintrag zugeordneten, kontextuellen Informationen eine(n) oder mehrere einer Syntax des ersten unstrukturierten Therapieeintrags, eines Erstellers des ersten unstrukturierten Therapieeintrags und/oder eines Formats des ersten unstrukturierten Therapieeintrags enthalten.

7. Verfahren nach Anspruch 6, wobei die zugeordneten Therapiemerkmale eines oder mehrere eines Arzneimittelwirkstoffes in einer Hierarchical Ingredient Code List (HICL), eines Arzneimittelnamens in einer National Drug Data File (NDDF), eines Therapienamens oder einer Klassifizierungsnummer umfassen.

8. Verfahren nach Anspruch 6, wobei die unstrukturierten Eintragsmerkmale einen oder mehrere eines unstrukturierten Arzneimittelwirkstoffes, eines unstrukturierten Arzneimittelnamens, eines unstrukturierten Therapienamens oder einer unstrukturierten Klassifizierungsnummer enthalten, und wobei das Verfahren weiterhin das Ermitteln einer ersten Wahrscheinlichkeit dahingehend, dass ein individuelles unstrukturiertes Eintragsmerkmal auf einen Satz zugeordneter Therapiemerkmale bezogen ist, sowie das Ermitteln einer zweiten Wahrscheinlichkeit dahingehend, dass das individuelle unstrukturierte Eintragsmerkmal auf eine spezifische Klasse zugeordneter Therapiemerkmale in dem Satz zugeordneter Therapiemerkmale bezogen ist, umfasst.

9. Verfahren nach Anspruch 6, das weiterhin das Ermitteln eines Zuverlässigkeitsfaktors umfasst, der für eine Korrelationsstärke zwischen dem ersten unstrukturierten Eintragsmerkmal und dem entsprechenden zugeordneten Therapiemerkmal indikativ ist.

10. Verfahren nach Anspruch 6, wobei eine Korrelation einen oder mehrere der folgenden Schritte umfasst, wonach:
Einheiten aus den unstrukturierten Therapieeinträgen herausgefiltert werden;
die Rechtschreibung von Wörtern in den unstrukturierten Therapieeinträgen korrigiert wird;
Wörter in den unstrukturierten Therapieeinträgen getrennt werden;
getrennte Präfix-Wörter in den unstrukturierten Therapieeinträgen kombiniert werden;
Rechtschreibfehler in den unstrukturierten Therapieeinträgen korrigiert werden;
Beschreibungen für Abkürzungen in den unstrukturierten Therapieeinträgen bereitgestellt werden; und/oder
nicht-semantische und/oder sinnwidrige Daten aus den unstrukturierten Therapieeinträgen entfernt werden.

## Revendications

1. Système (10) configuré pour corréler uniformément des caractéristiques d'entrées non structurées (212) dans des entrées de thérapie non structurées (202) avec des caractéristiques de thérapie associées (210) dans des informations de thérapie structurées (204), le système comprenant :
un ou plusieurs processeurs d'ordinateurs physiques (20) configurés par des instructions lisibles sur ordinateur pour :
obtenir des entrées de thérapie non structurées, les entrées de thérapie non structurées comprenant une première entrée de thérapie non structurée ;
identifier des caractéristiques d'entrées non structurées dans les entrées de thérapie non structurées individuelles ; et
corréler les caractéristiques d'entrées non structurées identifiées dans les entrées de thérapie non structurées avec des caractéristiques de thérapie associées correspondantes, cette corrélation étant basée sur des informations contextuelles associées aux entrées de thérapie non structurées dans lesquelles les caractéristiques d'entrées non structurées sont incluses de sorte que, à la suite de l'identification d'une première caractéristique d'entrée non structurée dans la première entrée de thérapie non structurée, la première caractéristique d'entrée non structurée soit corrélée avec une caractéristique de thérapie associée correspondante sur la base d'informations contextuelles associées à la première entrée de thérapie non structurée, les informations contextuelles associées à la première entrée de thérapie non structurée incluant un(e) ou plus d'un syntaxe de la première entrée de thérapie non structurée, d'un créateur de la première entrée de thérapie non structurée et/ou d'un format de la première entrée de thérapie non structurée.

2. Système selon la revendication 1, dans lequel les un ou plusieurs processeurs d'ordinateurs physiques sont configurés de sorte que les caractéristiques de thérapie associées comprennent un ou plusieurs d'un ingrédient de médicament dans une liste de codes d'ingrédients hiérarchique (HICL), d'un nom de médicament dans un fichier national de données de médicaments (NDDF), d'un nom de thérapie ou d'un numéro de classification.

3. Système selon la revendication 1, dans lequel les un ou plusieurs processeurs d'ordinateurs physiques sont configurés de sorte que les caractéristiques d'entrées non structurées comprennent un ou plusieurs d'un ingrédient de médicament non structuré, d'un nom de médicament non structuré, d'un nom de thérapie non structurée et d'un numéro de classification non structuré et dans lequel les un ou plusieurs processeurs d'ordinateurs physiques sont configurés pour déterminer une première probabilité qu'une caractéristique d'entrée non structurée individuelle soit rapportée à un ensemble de caractéristiques de thérapie associées et déterminer une seconde probabilité que la caractéristique d'entrée non structurée individuelle soit rapportée à une classe spécifique de caractéristiques de thérapie associées dans l'ensemble de caractéristiques de thérapie associées.

4. Système selon la revendication 1, dans lequel les un ou plusieurs processeurs d'ordinateurs physiques sont configurées pour déterminer un facteur de fiabilité indicatif d'une force de corrélation entre la première caractéristique d'entrée non structurée et la caractéristique de thérapie associée correspondante.

5. Système selon la revendication 1, dans lequel les un ou plusieurs processeurs d'ordinateurs physiques sont configurés de sorte que la corrélation comprenne une ou plusieurs des étapes suivantes :
la filtration d'unités venant des entrées de thérapie non structurées ;
la correction de l'orthographe de mots dans les entrées de thérapie non structurées ;
la séparation de mots dans les entrées de thérapie non structurées ;
la fusion de mots séparés de préfixes dans les entrées de thérapie non structurées ;
la correction de fautes d'orthographe dans les entrées de thérapie non structurées ;
la fourniture de descriptions pour des abréviations dans les entrées de thérapie non structurées ; et/ou
l'élimination de données non sémantiques et/ou de non-sens des entrées de thérapies non structurées.

6. Procédé pour corréler uniformément des caractéristiques d'entrées non structurées (212) dans des entrées de thérapie non structurées (202) avec des caractéristiques de thérapie associées (210) dans des informations de thérapie structurées (204) avec un système de corrélation (10), le système comprenant un ou plusieurs processeurs d'ordinateurs physiques (20), le procédé comprenant :
l'obtention avec les un ou plusieurs processeurs d'ordinateurs physiques d'entrées de thérapie non structurées, les entrées de thérapie non structurées comprenant une première entrée de thérapie non structurée ;
l'identification avec les un ou plusieurs processeurs d'ordinateurs physiques de caractéristiques d'entrées non structurées dans les entrées de thérapie non structurées individuelles ; et
la corrélation avec les un ou plusieurs processeurs d'ordinateurs physiques des caractéristiques d'entrées non structurées identifiées dans les entrées de thérapie non structurées avec des caractéristiques de thérapie associées correspondantes, cette corrélation étant basée sur des informations contextuelles associées aux entrées de thérapie non structurées dans lesquelles les caractéristiques d'entrées non structurées sont incluses de sorte que, à la suite de l'identification d'une première caractéristique d'entrée non structurée dans la première entrée de thérapie non structurée, la première caractéristique d'entrée non structurée soit corrélée avec une caractéristique de thérapie associée correspondante sur la base d'informations contextuelles associées à la première entrée de thérapie non structurée, les informations contextuelles associées à la première entrée de thérapie non structurée incluant un(e) ou plus d'un syntaxe de la première entrée de thérapie non structurée, d'un créateur de la première entrée de thérapie non structurée et/ou d'un format de la première entrée de thérapie non structurée.

7. Procédé selon la revendication 6, dans lequel les caractéristiques de thérapie associées comprennent un ou plusieurs d'un ingrédient de médicament dans une liste de codes d'ingrédients hiérarchique (HICL), d'un nom de médicament dans un fichier national de données de médicaments (NDDF), d'un nom de thérapie ou d'un numéro de classification.

8. Procédé selon la revendication 6, dans lequel les caractéristiques d'entrées non structurées comprennent un ou plusieurs d'un ingrédient de médicament non structuré, d'un nom de médicament non structuré, d'un nom de thérapie non structurée et d'un numéro de classification non structuré et dans lequel le procédé comprend en outre la détermination d'une première probabilité qu'une caractéristique d'entrée non structurée individuelle soit rapportée à un ensemble de caractéristiques de thérapie associées et la détermination d'une seconde probabilité que la caractéristique d'entrée non structurée individuelle soit rapportée à une classe spécifique de caractéristiques de thérapie associées dans l'ensemble de caractéristiques de thérapie associées.

9. Procédé selon la revendication 6, comprenant en outre la détermination d'un facteur de fiabilité indicatif d'une force de corrélation entre la première caractéristique d'entrée non structurée et la caractéristique de thérapie associée correspondante.

10. Procédé selon la revendication 6, dans lequel la corrélation comprend une ou plusieurs des étapes suivantes :
la filtration d'unités venant des entrées de thérapie non structurées ;
la correction de l'orthographe de mots dans les entrées de thérapie non structurées ;
la séparation de mots dans les entrées de thérapie non structurées ;
la fusion de mots séparés de préfixes dans les entrées de thérapie non structurées ;
la correction de fautes d'orthographe dans les entrées de thérapie non structurées ;
la fourniture de descriptions pour des abréviations dans les entrées de thérapie non structurées ; et/ou
l'élimination de données non sémantiques et/ou de non-sens des entrées de thérapies non structurées.
